Europäisches Patentamt

European Patent Office

Office européen des brevets

(19)

(11) Publication number: **0 089 676**
A2

(12) **EUROPEAN PATENT APPLICATION**

(21) Application number: 83102825.3

(22) Date of filing: 22.03.83

(51) Int. Cl.³: **C 12 N 15/00**
C 12 N 1/20, C 12 P 21/02
C 07 C 103/52, C 07 H 21/04
A 61 K 45/02
//C12R1/19, C12R1/125

(30) Priority: 24.03.82 PC T/JP82/00080
12.07.82 US 397384

(43) Date of publication of application:
28.09.83 Bulletin 83/39

(84) Designated Contracting States:
AT BE CH DE FR GB IT LI LU NL SE

(71) Applicant: Takeda Chemical Industries, Ltd.
27, Doshomachi 2-chome
Higashi-ku Osaka, 541(JP)

(72) Inventor: Kikuchi, Masakazu
4-16, Higashitokiwadai 7-chome
Toyono-cho Toyono-gun Osaka 563-01(JP)

(72) Inventor: Tsukamoto, Kyozo
1-402, 9 Uenohigashi 1-chome
Toyonaka Oaka 560(JP)

(72) Inventor: Kurokawa, Tsutomu
1-50, Suimeidai 1-chome
Kawanishi Hyogo 666-01(JP)

(74) Representative: von Kreisler, Alek, Dipl.-Chem. et al,
Deichmannhaus am Hauptbahnhof
D-5000 Köln 1(DE)

(54) Novel DNA and use thereof.

(57) Human immune interferon is produced by preparing a DNA which contains a base sequence coding for the human immune interferon polypeptide, transforming a host therewith, growing the host and recovering the same.

- 1 -

Novel DNA and Use Thereof

This invention relates to a DNA containing a base sequence coding for the human immune interferon polypeptide, a host transformed therewith, and a method of producing human immune interferon using said host, and various interferon products.

Background Art

Interferons (hereinafter sometimes abbreviated to "IFs") are proteins produced by cells of higher animals in response to the stimulation of a virus, nucleic acid, etc., and have antiviral, antitumor and other activities.

At present it is known that human IFs include three types, namely alpha, beta and gamma types, which are different in properties. The alpha and beta types are induced by a virus or nucleic acid and the gamma type is usually induced by mitogens.

Comparatively, the study of alpha type IF (hereinafter referred to an "IF-α") and beta type IF (hereinafter referred to as "IF-β") has considerably advanced, and the mechanisms of their production have been elucidated to a considerable extent. Furthermore, advances in gene manipulation techniques have made it possible to produce IF-α and IF-$\beta_1$ by growing Escherichia coli or to produce IF-$\alpha_1$ by growing a yeast; said IFs are thus obtainable in large amounts as physiologically active proteins [See D. V. Goeddel et al., Nature, 287, 411 (1980); E. Yelverton et al., Nucleic Acids Res., 9, 731 (1981); D. V. Goeddel et al.,

Nucleic Acids Res., 8, 4057 (1980); R. Hitzeman et al., Nature, 293, 717 (1981)]. Furthermore, attempts have been made to produce such IFs in amounts sufficiently large to enable commercial clinical use thereof.

Gamma type IF (hereinafter sometimes referred to as "IF-$\gamma$") is also called immune interferon (hereinafter sometimes referred to as "IFI") because it is produced by immunocompetent cells under those conditions which may induce blast-formation of lymphocytes or production of lymphokine. IFI has greater antiproliferative and antitumor activities than IF-$\alpha$ and IF-$\beta$, and therefore is more promising from the viewpoint of clinical application. However, no efficient production system therefor has been established because fresh lymphocytes are necessary. Furthermore, the possibility has been suggested that different cell species produce IFIs of different molecular species in different experimental systems.

On the other hand, since IFs are highly species-specific, human-derived IFs must be used in application to humans. However, mass production of human immune interferon has been so difficult that its application in clinical practice has thus far been impossible. Development of a technique capable of producing large amounts of highly pure human IFI in a simple manner and at low cost has been eagerly waited for.

Making use of gene manipulation techniques, the present inventors have engaged in developing a technique which enables production of the desired IFI by cloning the human IFI gene, incorporating the resulting recombinant DNA molecule into a host, thereby causing expression of the human IFI gene in said host and recovering the human IFI.

### Disclosure of the Invention

This invention provides a DNA containing a base sequence coding for the human immune interferon polypeptide, a host transformed with said DNA, a method of producing human immune interferon or a polypeptide equivalent thereto, which method comprises growing a host transformed with said DNA, and novel interferon products.

Thus, the DNA provided by the present invention is a DNA having the base sequence represented by the formula

```
               1
(5')   TGT TAC TGC CAG GAC CCA TAT GTA AAA GAA
       GCA GAA AAC CTT AAG AAA TAT TTT AAT GCA GGT CAT
       TCA GAT GTA GCG GAT AAT GGA ACT CTT TTC TTA GGC
       ATT TTG AAG AAT TGG AAA GAG GAG AGT GAC AGA AAA
       ATA ATG CAG AGC CAA ATT GTC TCC TTT TAC TTC AAA
       CTT TTT AAA AAC TTT AAA GAT GAC CAG AGC ATC CAA
       AAG AGT GTG GAG ACC ATC AAG GAA GAC ATG AAT GTC
       AAG TTT TTC AAT AGC AAC AAA AAG AAA CGA GAT GAC
       TTC GAA AAG CTG ACT AAT TAT TCG GTA ACT GAC TTG
       AAT GTC CAA CGC AAA GCA ATA CAT GAA CTC ATC CAA
                                           140
       GTG ATG GCT GAA CTG TCG CCA GCA GCT AAA ACA GGG
                   146
       AAG CGA AAA AGG AGT CAG ATG CTG TTT CGA GGT CGA
       AGA GCA TCC CAG -X (3')            (I)
```

wherein X is TAA, TGA or TAG.

The DNA of formula (I) codes for the polypeptide of the formula (N) Cys Tyr Cys Gln Asp Pro Tyr Val Lys Glu
Ala Glu Asn Leu Lys Lys Tyr Phe Asn Ala Gly His
Ser Asp Val Ala Asp Asn Gly Thr Leu Phe Leu Gly
Ile Leu Lys Asn Trp Lys Glu Glu Ser Asp Arg Lys
Ile Met Gln Ser Gln Ile Val Ser Phe Tyr Phe Lys
Leu Phe Lys Asn Phe Lys Asp Asp Gln Ser Ile Gln
Lys Ser Val Glu Thr Ile Lys Glu Asp Met Asn Val
Lys Phe Phe Asn Ser Asn Lys Lys Lys Arg Asp Asp
Phe Glu Lys Leu Thr Asn Tyr Ser Val Thr Asp Leu
Asn Val Gln Arg Lys Ala Ile His Glu Leu Ile Gln
Val Met Ala Glu Leu Ser Pro Ala Ala Lys Thr Gly
Lys Arg Lys Arg Ser Gln Met Leu Phe Arg Gly Arg
Arg Ala Ser Gln (C)                   (II)

or a polypeptide equivalent thereto in immunological or biological activities.

The above DNA (I) may have at the 5'-end thereof 5'ATG AAA TAT ACA AGT TAT ATC TTG GCT TTT CAG CTC TGC ATC
GTT TTG GGT TCT CTT GGC 3' (III) or
ATG (IV).

When the DNA (I) has the DNA of formula (III) at the 5' end, it codes not only for the polypeptide (II) but also for the polypeptide having Met Lys Tyr Thr Ser Tyr Ile Leu Ala Phe Gln Leu Cys Ile Val Leu Gly Ser Leu Gly added to the N-terminus or a polypeptide equivalent thereto in activities. When the DNA (I) has the DNA of formula (IV) at the 5'-end, it codes not only for the polypeptide (II) but also for the polypeptide having Met added to the N-terminus or a polypeptide equivalent thereto in activities.

The DNA (I) is preferably connected at the site of downstream from a promoter. Suitable promoters therefor include the tryptophan (trp) promoter, the bacteriophage lambda $P_L$ promoter or other known promoters such as the lactose (lac) promoter, and the protein chain elongation factor Tu (tufB) promoter. Preferable among them are the trp promoter and the $P_L$ promoter. The $P_L$ promoter is a very strong promoter that can be efficiently and conveniently controlled by the λ cI repressor. The gene encoding the repressor carries a mutation, cIts2 or cIts857, which renders the repressor temperature-sensitive. At 30° the repressor functions normally, and from about 37°C to about 42°C it is inactivated. Thus the $P_L$ promoter is repressed (turned-off) at 30°C and derepressed (turned-on) at 42°C. This feature is desirable herein in that the gene product of interest may be toxic to the cell or that if present in large quantity it would be detrimental to cell growth. The ability to control the $P_L$ promoter allows one to grow the culture at about 30°C to about 36°C without expressing the gene product and at an optimum time, shift the temperature from about 37°C to about 42°C to produce the desired gene product.

In general formula (I), X, which is an oligodeoxyribonucleotide, is preferably TAA.

The symbols as used herein in the specification, drawings and claims have the meanings shown in Table 1.

## Table 1

| | | |
|---|---|---|
| DNA | = | deoxyribonucleic acid |
| A | = | adenine |
| T | = | thymine |
| G | = | guanine |
| C | = | cytosine |
| RNA | = | ribonucleic acid |
| dATP | = | deoxyadenosine triphosphate |
| dTTP | = | deoxythymidine triphosphate |
| dGTP | = | deoxyguanosine triphosphate |
| dCTP | = | deoxycytidine triphosphate |
| ATP | = | adenosine triphosphate |
| EDTA | = | ethylenediamine tetraacetate |
| SDS | = | sodium dodecyl sulfate |
| Gly | = | glycine |
| Ala | = | alanine |
| Val | = | valine |
| Leu | = | leucine |
| Ile | = | isoleucine |
| Ser | = | serine |
| Thr | = | threonine |
| Cys | = | cysteine |
| Met | = | methionine |
| Gly | = | glutamic acid |
| Asp | = | aspartic acid |
| Lys | = | lysine |
| Arg | = | arginine |
| His | = | histidine |
| Phe | = | phenylalanine |
| Tyr | = | tyrosine |
| Trp | = | trypotophan |
| Pro | = | proline |
| Asn | = | asparagine |
| Gln | = | glutamine |

The DNA of the present invention having the base sequence represented by the formula (I) can be produced, for example, by

(a) growing human IFI-secreting cells, for example, human peripheral blood lymphocytes,

(b) isolating from the cells a messenger (m) RNA coding for human IFI,

(c) synthesizing a single-stranded complementary (c) DNA with the use of said messenger RNA and reverse transcriptase,

(d) converting said (c) DNA to a double-stranded DNA,

(e) inserting said double stranded DNA into a plasmid,

(f) transforming a host, e.g. Escherichia coli or Bacillus subtilis, with said recombinant plasmid,

(g) isolating a plasmid containing the objective DNA from the culture of said host,

(h) if desired, excising out the cloned DNA from the plasmid, and

(i) if desired, connecting said cloned DNA at the site of downstream from a promoter in a vehicle.

The human peripheral blood lymphocytes to be used in the practice of the invention may be either uninduced cells or induced cells.

The IFI inducer to be used in practicing the invention may be any agent capable of inducing human lymphocytic cells to produce IFI and includes, among others, phytohemaggutinin (PHA), Concanavalin A (ConA), staphylococcal enterotoxin A (SEA), neuraminidase-galactose oxidase (NAGO), 12-0-tetradecanoylphorbol-13-acetate (TPA) and combinations thereof.

Induction of IFI can be effected by growing the cells in a per se known medium, such as RPMI-1640 or MEM medium, preferably in RPMI-1640 medium containing foetal bovine serum. The cultivation is carried out at a temperature of 35-39°C, desirably 36-38°, for 12-72 hours, desirably 22-26 hours. The cells are collected (by a per se known method) and, after addition, for example, of guanidine thiocyanide, bentonite, heparin, polyvinyl sulfate, aurintricarboxylic acid, vanadium complex, diethyl pyrocarbonate (DPC) or sodium dodecyl sulfate (SDS) as the RNase inhibitor, lysed for RNA extraction by adding sodium-N-lauroylsarcosinate, Tween 80 or Nonidet P-40, for example. After repeated extraction as necessary by adding phenol, phenol-chloroform or the like to the RNA-containing extract, RNAs are collected by precipitation with ethanol. Since many of mRNAs present in the cytoplasm of eukaryotic cells are known to contain a poly(A) sequence at the 3' end, poly(A)$^+$ RNAs are collected with the use of oligo (dT) cellulose, poly(U) sepharose or the like and fractionated by the sucrose density gradient centrifugation method. Aliquots of each fraction obtained are injected into oocytes of Xenopas laevis (a frog species) for translation. [J. B. Gurdon, et al., Nature, 233, 177 (1971)]. The produced protein is assayed for antiviral activity. In this manner, the mRNA for IFI can be obtained. The mRNA can also be purified by formamide-polyacrylamide gel electrohoresis or by agarose gel electrophoresis or by agarose gel electrophoresis using glyoxal.

Using the thus-obtained mRNA as the template, the single-stranded complementary DNA (cDNA) chain is synthesized by a per se known method, for instance, with the use of a reverse transcriptase. The cDAN is then converted to the double-stranded DNA [T. Maniatis et al., Cell, 8, 163 (1976)].

This DNA is inserted, for example, into the plasmid pBR322, e.g., at the PstI or SphI restriction endonuclease cleavage site, for example by the dG-dC or dA-dT homopolymer tailing method described in T. S. Nelson [Methods in Enzymology, 68, 41 (1979), Academic Press Inc., New York]. The recombinant plasmid is then introduced, for example, into Escherichia coli χ1776, by transformation. The transformants can be selected as the tetracycline-resistant or ampicillin-resistant colonies. Separately, a probe oligonucleotide having a base sequence supposedly corresponding to at least part of the amino acid sequence of the IFI polypeptide is synthesized chemically and labeled with $^{32}P$. Using the labeled oligonucleotide as the probe, the desired clones are secondarily screened out from among the already-obtained tetracycline- or ampicillin-resistant transformants, for example by the per se known colony hybridization method [M. Grunstein and D. S. Hogness, Proc. Natl. Acad. Sci. USA, 72, 3961 (1975)].

For confirming the presence of the IFI gene, the base sequence of the clones showing the positive colony hydridization result is determined, for example, by the Maxam-Gilbert method [A. M. Maxam and W. Gilbert, Proc. Natl. Acad. Sci. USA, 74, 560 (1977)] or the dideoxynucleotide synthetic chain termination method using phage M13 [J. Messing et al., Nucleic Acids Res., 9, 309 (1981)]. The whole or part of the IFI gene may also be excised from the clones obtained and then connected downstream from an adequate promoter and SD (Shine-Delgarno) sequence, for introduction into an adequate host.

The promoter includes the above-mentioned promotors and the host includes such bacteria as Escherichia coli or Bacillus subtilis, and other microorganisms, such as the yeast Saccharomyces cerevisiae. Preferred as the host is Escherichia coli, such as E. coli strains 294 or W3110, especially E. coli 294.

Escherichia coli 294 is a known bacterial strain [K. Backman et al., Proc. Natl. Acad. Sci. USA, 73, 4174 (1976)] and is also on deposit in the Institute for Fermentation, Osaka (Deposit No. IFO-14171).

The transformation of a host with the DNA of the invention is carried out, for example, by the known method of S. N. Cohen, et al. [Proc. Natl. Acad. Sci. USA, 69, 2110 (1972)].

The compound of the formula:

```
Y-Cys Tyr Cys Gln Asp Pro Tyr Val Lys Glu Ala Glu
    Asn Leu Lys Lys Tyr Phe Asn Ala Gly His Ser Asp
    Val Ala Asp Asn Gly Thr Leu Phe Leu Gly Ile Leu
    Lys Asn Trp Lys Glu Glu Ser Asp Arg Lys Ile Met
    Gln Ser Gln Ile Val Ser Phe Tyr Phe Lys Leu Phe
    Lys Asn Phe Lys Asp Asp Gln Ser Ile Gln Lys Ser
    Val Glu Thr Ile Lys Glu Asp Met Asn Val Lys Phe
    Phe Asn Ser Asn Lys Lys Lys Arg Asp Asp Phe Glu
    Lys Leu Thr Asn Tyr Ser Val Thr Asp Leu Asn Val
    Gln Arg Lys Ala Ile His Glu Leu Ile Gln Val Met
    Ala Glu Leu Ser Pro Ala Ala Lys Thr Gly Lys Arg
    Lys Arg Ser Gln Met Leu Phe Arg Gly Arg Arg Ala
    Ser Gln                    (II')
```

wherein Y is (a) hydrogen, (b) Met or (c) Met Lys Tyr Thr Ser Tyr Ile Ala Phe Gln Leu Cys Ile Val Leu Gly Ser Leu Gly can be produced, for example, by growing thus obtained host, accumulating compound (II') in the culture broth and recovering the same.

The host is grown in a _per se_ known medium.

The medium is, for example, M9 medium containing glucose and casamino acids [J. Miller, Experiments in Molecular Genetics, 431-433, Cold Spring Harbor Laboratory, New York, 1972]. For efficient working of the promoter, a derepressing agent such as β-indolylacrylic acid may be added as necessary.

The incubation is carried out generally at 15-43°C for 3-24 hours, if necessary with aeration and/or stirring.

When a transformant having a λ cI repressor and an expression vector containing the $P_L$ promoter is used, lower temperatures are used for incubation, i.e., approximately 30° to about 36°C and the λ cI repressor may advantageously be inactivated under incubation at about 37° to 42°C.

After the incubation, the cells are collected by a known method and, after suspending in a buffer solution, for instance, lysed, for example, by ultrasonic treatment, by lysozyme, and/or freezing and thawing, or other methods [e.g., Gunsalus, "Extraction of Enzymes from Microorganisms", Methods in Enzymology I:51 (1955). The supernatant is recovered by centrifugation.

The isolation of human IFI from said supernatant may be carried out by commonly known methods for protein purification.

The IFI, or polypeptide equivalent thereto in immunological or biological activities, as produced in accordance with the invention has immunological and biological activities equivalent to those of the IFI species produced by previously known methods and can be used in the same manner and for the same purposes as the known IFI species.

IFI has exhibited antiviral, antitumor, growth inhibition and immunosuppression activity. The compound can be mixed with a sterile pharmaceutically acceptable carrier such as sterile water, human serum albumin, (HSA), normal saline solution, or other carriers well known in the art, and administered orally or topically. Daily dosages for intravenous treatment range from about 10 million units to about 100 million units per day for normal adults, preferably about 50 to 60 million units per day. The pharmaceutical product may contain other pharmaceutically acceptable ingredients, such as salts, diluents, adjuvants, other carriers, buffers, binders, surface active agents, preservatives, etc. Preparations for parenteral use could be provided as an ampoule of a sterile solution or suspension of the product with water or another pharmaceutically acceptable liquid as the carrier therefore, or an ampoule of sterile powder (preferably obtained by lyophilization of a solution of IFI) for dilution with pharmaceutically acceptable liquid.

Moreover, the product of the present invention may contain other pharmaceutically active components which are suitably administerable with IFI, including for example lymphocyte or fibroblast interferon, in amounts ranging from about 1-99%, based on the amount of IFI.

## Brief Description of the Drawings

Fig. 1 shows the restriction enzyme cleavage map of the plasmid pHIT3709 as obtained in Example 1 (vii) hereof, the portion ▨▨▨▨ indicating the portion coding for the peptide supposed to be the signal peptide and the portion ▨▨▨▨ indicating the portion coding for the IFI polypeptide. Fig. 2 shows the primary structure (base sequence) of the plasmid pHIT3709 as obtained in Example 1 (vii). Fig. 3 shows the construction scheme for Example 2.

Fig. 4 is a partial restriction map of a 1200 bp Bgl II - Bam H l fragment containing a $P_L$ promoter. Fig. 5 illustrates the construction of an expression vector pRC14, which contains the lambda $P_L$ promotor on a 350 bp insert.

Fig. 6 illustrates the scheme used to isolate the 82 bp fragment containing the SD sequence for the int gene of bacteriophage lambda for construction of expression vector pRC15.

Fig. 7 illustrates the construction of the expression vector pRC15. Fig. 8 illustrates the construction of expression vectors pRC21 and pRC22. Figure 9 illustrates the construction of expression vector pRC23 with a $P_L$ promoter and a synthetic RBS.

Figure 10 shows the scheme used to insert the gene coding for immune interferon into pRC22, and the sequence of the promoter-gene junction.

Fig. 11 illustrates the insertion of the immune interferon gene into expression vector pRC23, and the sequence of the promoter-gene junction and its modification.

## Example 1

(i)   Isolation of mRNA coding for IFI

Lymphocytes prepared from human peripheral blood were incubated in RPMI-1640 medium (containing 10% foetal bovine serum) containing 15 ng/ml of 12-0-tetradecanoylphorbol-13-acetate (TPA) and 40 µg/ml of Concanavalin A at 37°C for IFI induction. After 24 hours of incubation, the thus- induced human lymphocytes ($1 \times 10^{10}$ cells) were destroyed and denatured in a thioguanidine solution (5 M guanidine thiocyanate, 5% mercaptoethanol, 50 mM Tris HCl (pH 7.6), 10 mM EDTA) in a Teflon homogenizer. Then sodium N-lauroyl sarcosinate was added in the concentration of 4% and the mixture, after homogenization, was layered on 6 ml of 5.7 M cesium chloride solution (5.7 M cesium chloride, 0.1 M EDTA) and centrifuged at 15°C and 24,000 rpm for 30 hours using a Beckman SW27 rotor to give an RNA precipitate.

This RNA precipitate was dissolved in 0.25% N-lauroyl sarcosinate and then precipitated with ethanol to give 8.3 mg of RNA. This RNA was allowed to be adsorbed, in a high concentration salt solution (0.5 M NaCl, 10 mM Tris HCl (pH 7.6), 1 mM EDTA, 0.3% SDS), on an oligo (dT) cellulose column and the poly (A)-containing mRNA was eluted with a low concentration salt solution (10 mM Tris HCl (pH 7.6), 1 mM EDTA, 0.3% SDS). There was collected 700 µg of mRNA.

This mRNA was again precipitated with ethanol, then dissolved in 0.2 ml of a solution (10 mM Tris HCl (pH 7.6), 2 mM EDTA, 0.3% SDS), treated at 65°C for 2 minutes and fractioned to 22 portions by 10-35% sucrose density gradient centrifugation at 20°C and 25,000 rpm for 21 hours using a Beckman SW 27 rotor. Aliquots of each fraction were injected into Xenopas laevis oocytes and the proteins synthesized were assayed for interferon activity, i.e., assayed for antiviral activity as assayed by the inhibition test of the cytopathic effect of the vesicular stomatitis virus against human amnion-derived WISH cells [W. E. Stewart, The Interferon System 11-26, Springer-Verlag, New York (1979)]. In this manner, it was revealed that fraction 12 (the sedimentation coefficient being 12-14S) had an activity of 195 units (international IFN units) per microgram of RNA. The mRNA in the thus-obtained fraction 12 weighed about 20 µg.

(ii)   Synthesis of single-stranded DNA

Using the above mRNA and a reverse transcriptase, 100 μl of a reaction mixture (5 μg of mRNA, 50 μg of oligo (dT), 100 units of reverse transcriptase, 1 mM each of dATP, dCTP, dGTP and dTTP, 8 mM $MgCl_2$, 50 mM KCl, 10 mM dithiothreitol and 50 mM Tris-HCl (pH 8.3) were incubated at 42°C for an hour, then deproteinized with phenol, and treated with 0.1 N NaOH at 70°C for 20 minutes for degradation of RNA.

(iii) Synthesis of double-stranded DNA

The thus-synthesized single-stranded complementary DNA was subjected to reaction in 50 μl of a reaction mixture (the same mixture as above-mentioned except that the mRNA and oligo (dT) were absent) at 42°C for 2 hours for synthesizing the double-stranded DNA.

(iv)   Addition of dC tails

The above double-stranded DNA was treated with nuclease Sl in 50 μl of a reaction mixture (double stranded DNA, 0.1 M sodium acetate (pH 4.5), 0.25 M NaCl, 1.5 mM $ZnSO_4$, 60 units Sl nuclease) at room temperature for 30 minutes. The reaction mixture was deproteinized with phenol, the DNA was precipitated with ethanol and subjected to terminal transferase reaction in a mixture of double-stranded DNA, 0.14 M potassium cacodylate, 2 mM dithiothreitol, 1 mM $CoCl_2$, 0.15 mM dCTP, 30 units terminal transferase in 0.3 M Tris (base) (pH 7.6), at 37°C for 3 minutes for addition to the double-stranded DNA of about 20 deoxycytidine chains at each 3'-end of the DNA.

This series of reactions gave about 300 ng of a double-stranded deoxycytidine-chain-containing DNA.

(v)   Cleavage of Escherichia coli plasmid and addition of dG tails

Separately, 10 μg of Escherichia coli plasmid pBR322 DNA was treated with restriction enzyme PstI in 50 μl of a reaction mixture containing 10 μg pBR322 DNA, 50 mM NaCl, 6 mM $MgCl_2$, 6 mM 2-mercaptoethanol, 100 μg/ml bovine

serum albumin, and 20 units PstI in 6 mM Tris HCl (pH 7.4), at 37°C for three hours, for cleavage at the one PstI recognition site present in the pBR322 DNA. The reaction mixture was then deproteinized with phenol and the DNA was further treated with terminal transferase in 50 µl of a reaction mixture containing 10 µg DNA, 0.14 M potassium cacolylate, 2 mM dithiothreitol, 1 mM $CoCl_2$, 0.15 mM dGTP, and 30 units terminal transferase in 0.3 M Tris (base) (pH 7.6) at 37°C for 3 minutes for addition of about 8 deoxyguanosine residues at each 3'-end of the above plasmid pBR322 DNA.

(vi) Annealing of cDNA and transformation of Escherichia coli

The annealing was effected by heating 0.1 µg of the thus-obtained dC-tailed synthetic double-stranded DNA and 0.5 µg of the above dG-tailed plasmid pBR322 in a solution containing 0.1 M NaCl, 50 mM Tris HCl (pH 7.6) and 1 mM EDTA at 65°C for 2 minutes and then at 45°C for 2 hours, followed by gradual cooling. The transformation of Escherichia coli χ1776 was performed by the method of Enea et al. [J. Mol. Biol., 96, 495 (1975)].

(vii) Isolation of plasmid containing cDNA

About 8500 tetracycline-resistant colonies were thus isolated, and the DNA of each colony was fixed to a nitrocellulose filter [M. Grunstein and D. S. Hogness, Proc. Natl. Acad. Sci. USA, 72 3961 (1975)].

Separately, based on the amino acid sequence of IFI as reported by D. V. Goeddel et al. [Nature, 295, 503 (1982)], two base sequences 5' TCCTGGCA$_G^A$TA$_G^A$C 3' and 5' AC$_A^G$TTCAT$_A^G$TC$_C^T$TC$_C^T$T 3', presumably corresponding to amino acids Nos. 1-5 (Cys·Tyr·Cys·Gln·Asp) and amino acids Nos. 77-82 (Lys·Gln·Asp·Met·Asn·Val) of said IFI sequence, respectively, were chemically synthesized by the triester method described in R. Crea et al. [Proc. Natl. Acad. Sci USA, 75, 5765 (1978)]. These oligonucleotides were treated with T4 polynucleotide kinase in 50 µl of a reaction

mixture (0.2 μg oligonucleotide, 10 mM $MgCl_2$, 10 mM mercaptoethanol, 50 μCi $\gamma-^{32}P$-ATP, and 3 units T4 polynucleotide kinase in 50 mM Tris HCl (pH 8.0), at 37°C for an hour. These oligonucleotides thus labeled with $^{32}P$ at the 5'-end were used as probes and annealed with the DNA on the above-mentioned nitrocellulose filter by the method of Lawn et al [Nucleic Acids Res., 9, 6103 (1981)]. Four colonies reactive to the above two oligonucleotide probes were isolated using autoradiography.

Plasmid DNAs were isolated from the bacterial cells of each of these colonies by the method of Birnboim and Doly [Nucleic Acids Res. 7, 1513 (1979)]. The inserts in the plasmid DNAs were excised with the PstI restriction enzyme. From among the isolated plasmids, the one containing the longest cDNA insert was chosen and named "pHIT3709."

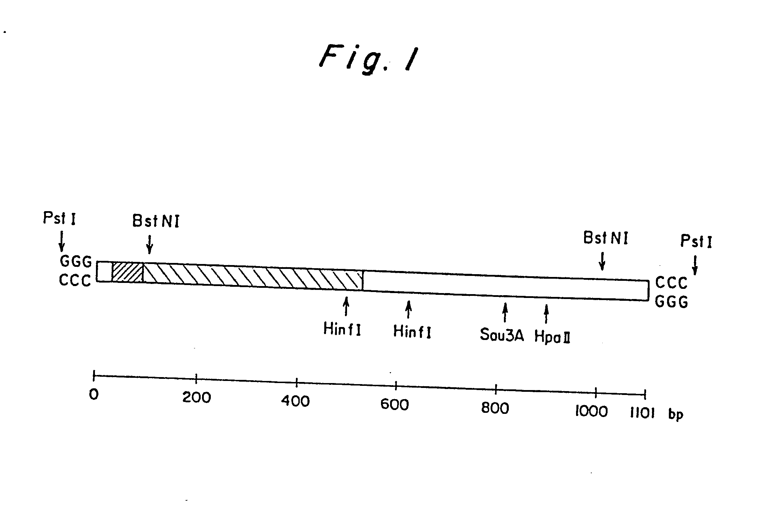

The restriction enzyme map of this plasmid is shown in Fig. 1.

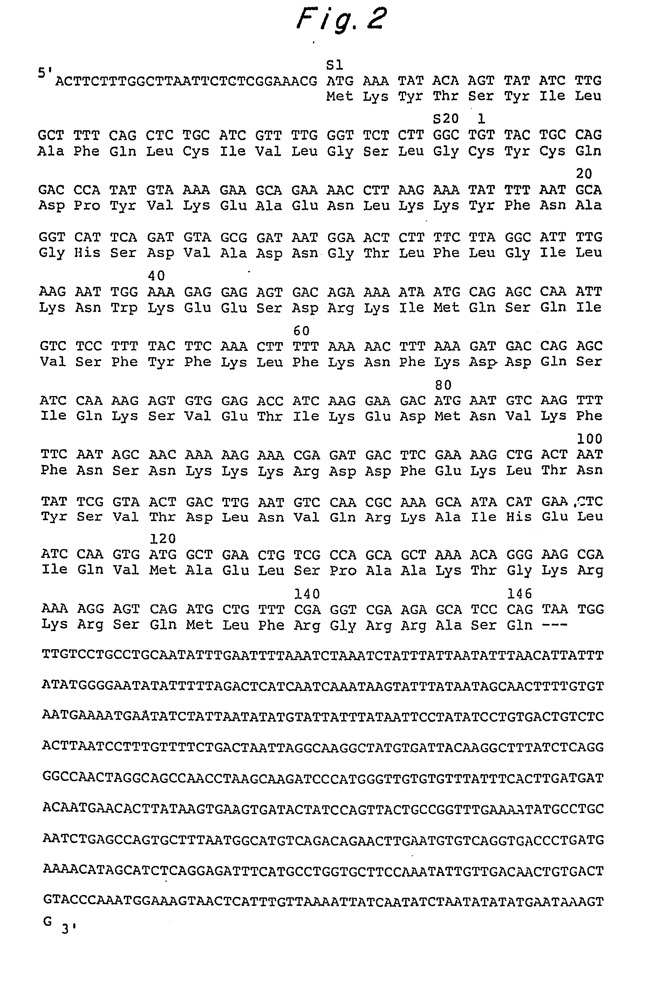

The primary structure (base sequence) of the cDNA sequence inserted in the pHIT3709 plasmid was then determined by the dideoxynucleotide synthetic chain termination method and by the Maxam-Gilbert method. Said primary structure was as shown in Fig. 2.

This primary structure is in agreement with that of IFI cDNA as reported by Gray et al. [Nature, 295, 503-508 (1982)] except that the former differs from the latter in one codon; namely the former's codon for the No. 140 amino acid is CGA, while the latter's is CAA for Gln. The protein determined by this base sequence presumably consists of 166 amino acids whose synthesis is initiated from the No. 30 nucleotide, namely the ATG codon, which is the signal for the start of protein synthesis. The first 20 amino acids probably constitute a signal peptide. The amino acid sequence, too, is different from the IFI reported by Gray et al. with regard to the No. 140 amino acid (Arg in place of Gln).

From the above-mentioned primary structure, it is clear that this plasmid has the entire coding region for the IFI protein. This fact permits making hosts such as Escherichia coli produce immune interferon by transfering the DNA sequence inserted in this plasmid to another expression plasmid.

Example 2

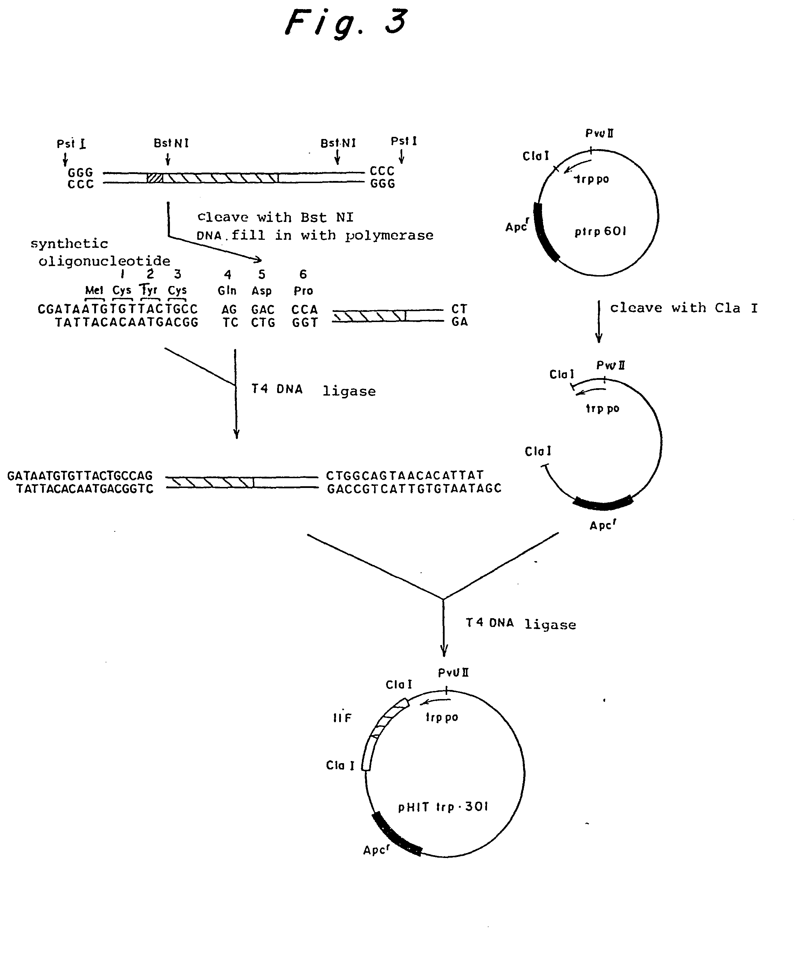

The insert in the pHIT3709 plasmid as obtained in Example 1 was excised from the No. 100 nucleotide thereof with the BstNI restriction enzyme (cf. Fig. 2 and Fig. 3) and, after filling in of the sticky ends with DNA polymerase I large fragment (also called Klenow's DNA polymerase I), ligated, with the use of T4 DNA ligase, with the oligonucleotide adapter:

         C G A T A A T G T G T T A C T G C C
             T A T T A C A C A A T G A C G G

which was chemically synthesized by the above-mentioned triester method and contains the protein synthesis start codon ATG.

A second plasmid (ptrp601) containing pBR322 and the tryptophan promoter portion of Escherichia coli (i.e., the promoter- and operator-containing DNA fragment), containing 276 base pairs, was constructed as the expression plasmid, using the method of G. N. Bennett et al. [J. Mol. Biol. 121, 113 (1978)].

This plasmid ptrp601 was cut with the ClaI restriction enzyme, and the IFI gene with the above-mentioned adapter ligated thereto was inserted into the plasmid vector downstream from the tryptophan promoter, using T4 DNA ligase. An IFI expression plasmid pHIT·trp301 was thus constructed (Fig. 3).

A bacterial strain containing this plasmid pHIT· trp301 was obtained by transforming Escherichia coli 294 with that plasmid by the method of Cohen et al. supra.

Example 3

The bacterial strain containing the IFI expression plasmid as obtained in Example 2 is grown in M9 medium containing 0.5% glucose and 0.5% casamino acids at 37°C for 6 hours. The cells are then collected and suspended in 0.15 M NaCl solution in 0.02 M phosphate buffer (pH 6.8) and lysed by ultrasonic treatment. Centrifugation gives a supernatant. The interferon activity of this supernatant can be measured by assay on WISH cells in accordance with the method mentioned in Example 1(i) supra, and described by W. E. Steward, supra.

Example 4

The bacterial strain containing the IFI expression plasmid, pHIT·trp301, as obtained in Example 2 was grown in M9 medium containing 0.5% glucose and 0.5% casamino acids at 37°C for 6 hours under shaking.

Indole acrylic acid was added to a final concentration of 20 µg/ml and incubation was further continued at 37°C for 3 hours under shaking.

A 10 ml sample was taken, the cells were collected by centrifugation and resuspended in 0.1 ml of 50 mM Tris (pH 7.4) containing 10 percent sucrose, and the suspension was quick-frozen in a dry ice/ethanol bath. Then, the cells were thawed at 20°C, and transferred to an ice bath. To the cell suspension NaCl was added to a concentration of 100 mM, EDTA to 10 mM, spermidine to 20 mM, and lysozyme to 200 µg/ml. The mixture was kept on ice for 45 minutes, then incubated at 37°C for 2 minutes. Lysis was apparent by the increase in viscosity of the suspension. Cell debris was removed by centrifugation and the supernatant was assayed for IFI anti-viral activity on WISH cells. This experiment resulted in a yield of 1280 units of IFI activity per ml of cell extract.

The following illustrative embodiments illustrate the expression of IFI utilizing the $\lambda P_L$ promoter system together with the IFI coding DNA segment of the present invention.

## Example 5

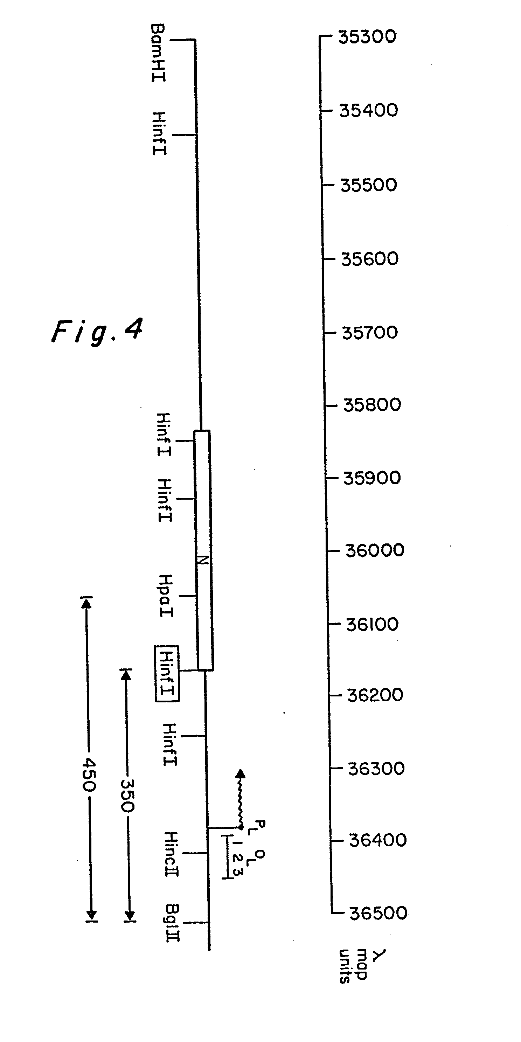

In order to isolate a 350 base pair (bp) fragment containing the $\lambda$ $P_L$ promoter, 250 µg of $\lambda$ cI857 Sam7 DNA (Miles Laboratories) was digested with restriction endonucleases BamHl and Bgl II, and the products were separated on agarose gel by electrophoresis. A 1200 bp fragment containing the $P_L$ promoter was isolated from the gel (see Fig. 4).

This 1200 bp fragment was then digested completely with HpaI and partially digested with Hinf I and a 350 bp fragment was isolated from a 5% polyacrylamide gel. Digestion with HpaI eliminated 2 HinfI partial fragments of about 350 bp that would have otherwise contaminated the desired fragment.

This 350 bp fragment contains the $P_L$ promoter and the $O_L$ operator sites ($O_{L1}$, $O_{L2}$, $O_{L3}$) to which the $\lambda$ cI repressor binds. The HinfI site, which occurs between the Shine-Dalgarno (SD) sequence of the $\lambda$ N-gene and the initiating codon for the N gene (see Fig. 4), allows for the construction of hybrid ribosome-binding sites for the purpose of expressing foreign genes in E. coli. This HinfI site may also be converted to an EcoRl site.

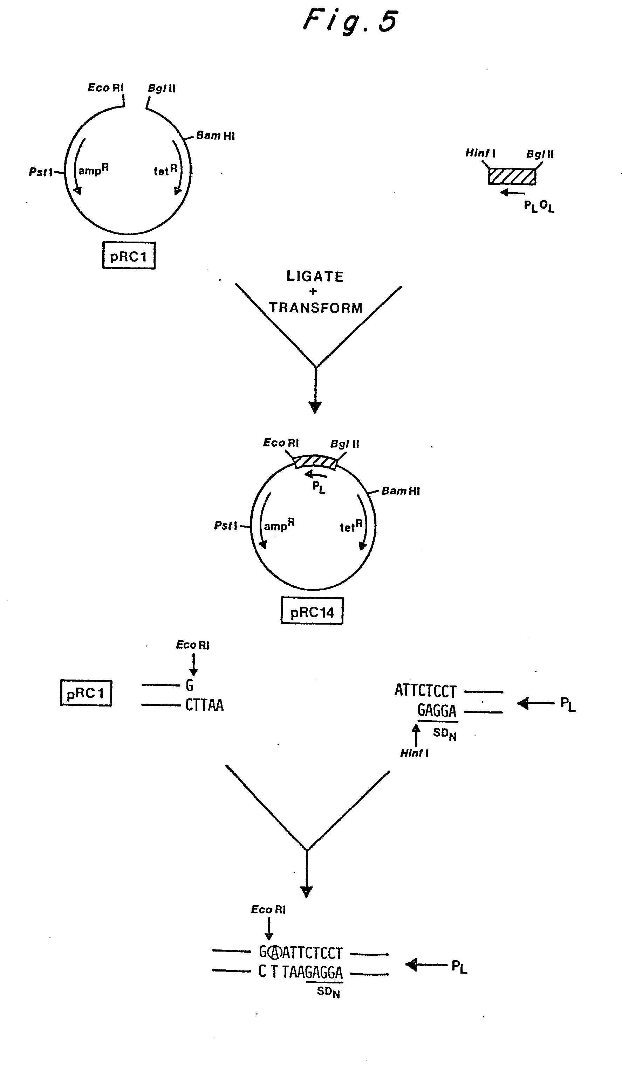

The 350 bp Bgl II - HinfI fragment was cloned into plasmid pRC1 which had been digested with EcoRl and Bgl II. pRC1 is a derivative of pBR322 that contains a Bgl II site adjacent to the EcoRl site (see Fig. 5). Figure 5 shows the sequences of the joining terminal and indicates how the EcoRl terminus of the vector can join to the HinfI terminus of the fragment, thus reconstituting the EcoRl site. (The circles A in Figure 5 was filled-in in vivo.) The resulting recombinant plasmid was designated pRC14.

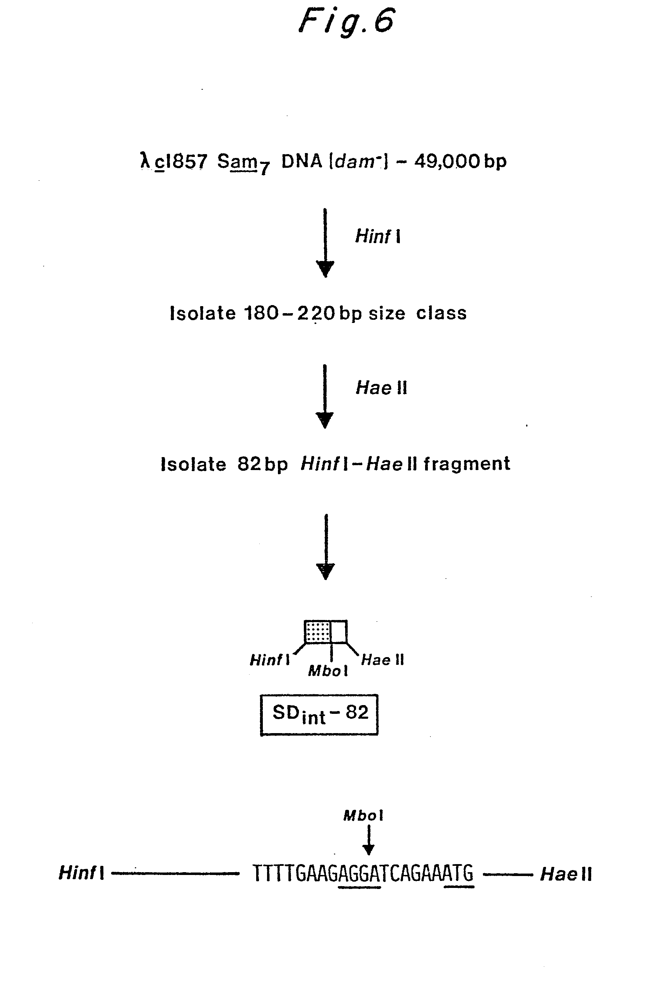

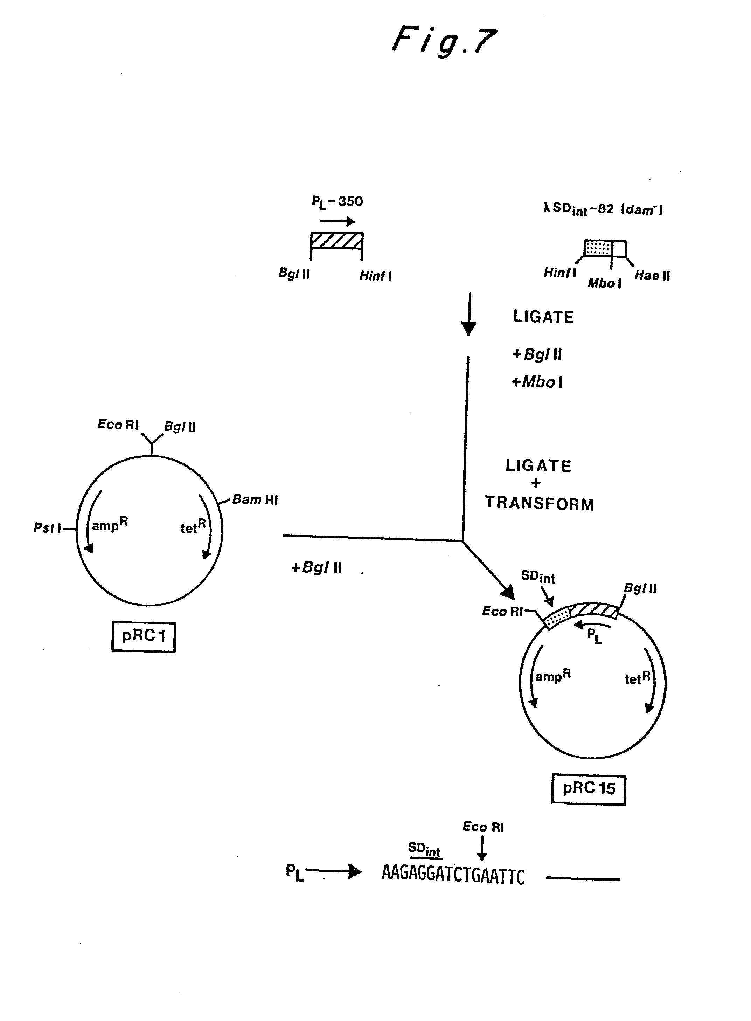

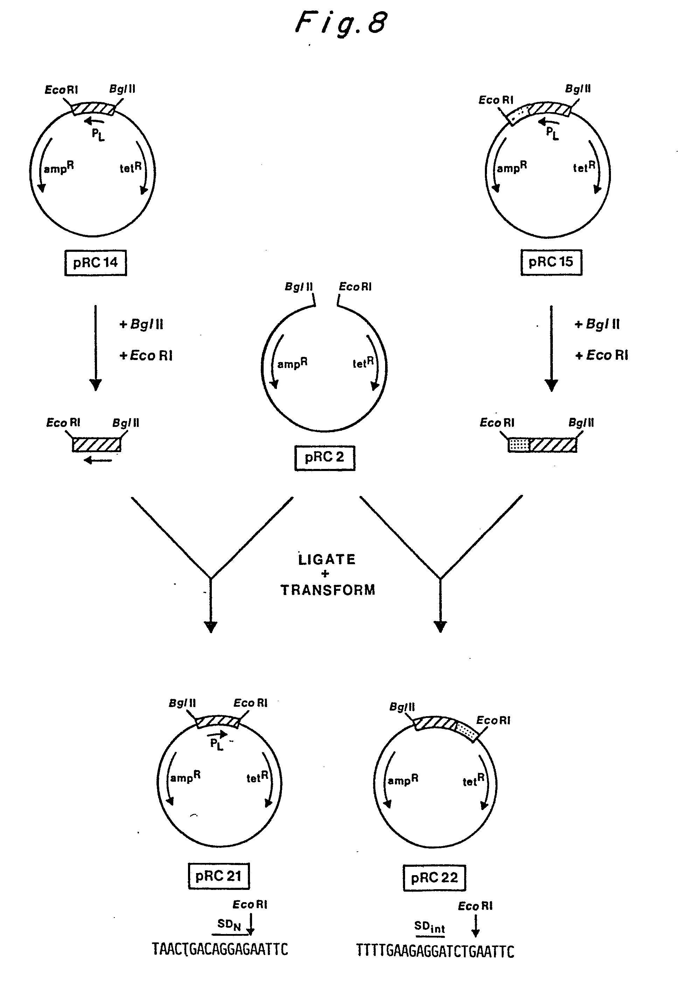

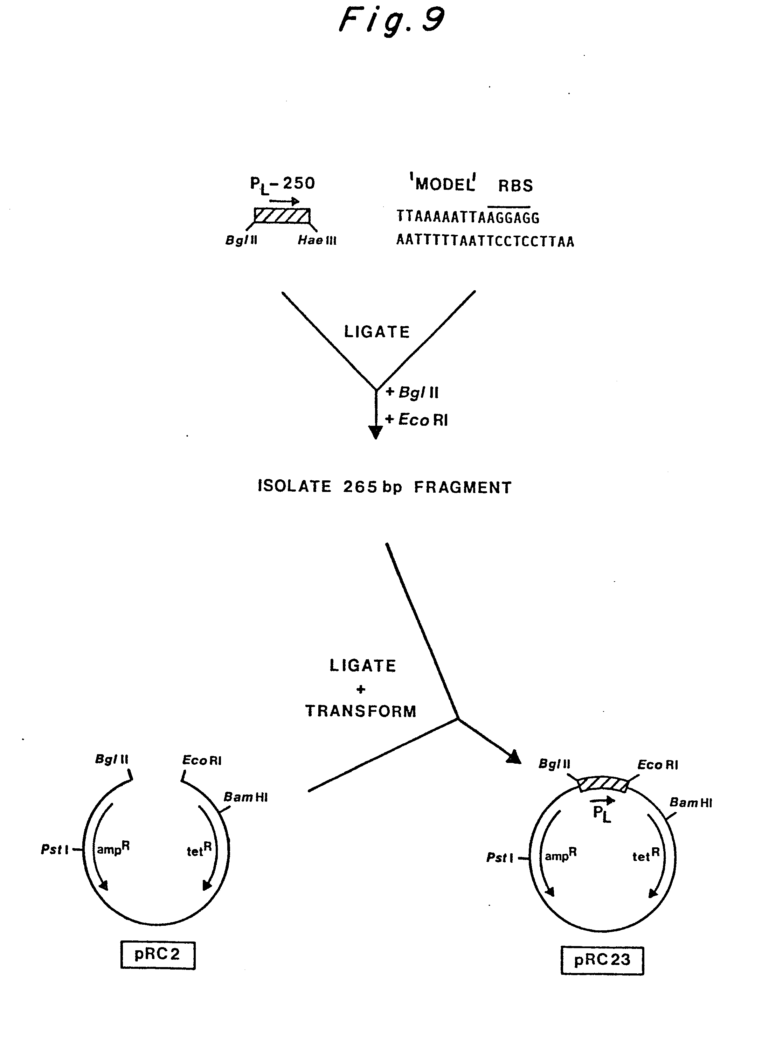

Using the same techniques, a number of other expression vectors using the $P_L$ promoter were also constructed (Figs. 6, 7, 8, and 9). pRC15 contains, in addition to the 350 bp Bgl II - HinfI fragment described above, a 55 base pair fragment) (Hinf [-$_{Mboi}$]) that contains the SD sequence of the λ int gene (see Figs. 6 and 7). pRC21 and pRC22 are analogous to pRC14 and pRC15, respectively. The principle difference between these plasmids is the orientation of the inserted P$_L$ - containing fragment, and thus the direction of transcription (see Fig. 8). pRC23 was constructed by ligating synthetic oligonucleotides containing a "consensus" RBS (Scherer, et al., Nucleic Acids Research, 8, 3895 (1980) to a 250 bp Bgl II - Hae III fragment containing the P$_L$ promoter, and inserting the ligation product into pRC2 as shown in Figure 9.

P$_L$-expression vectors were also constructed containing the Immune Interferon (IFI) gene from plasmid pHIT3709, from Example 1, supra, as shown in the following examples.

Example 6

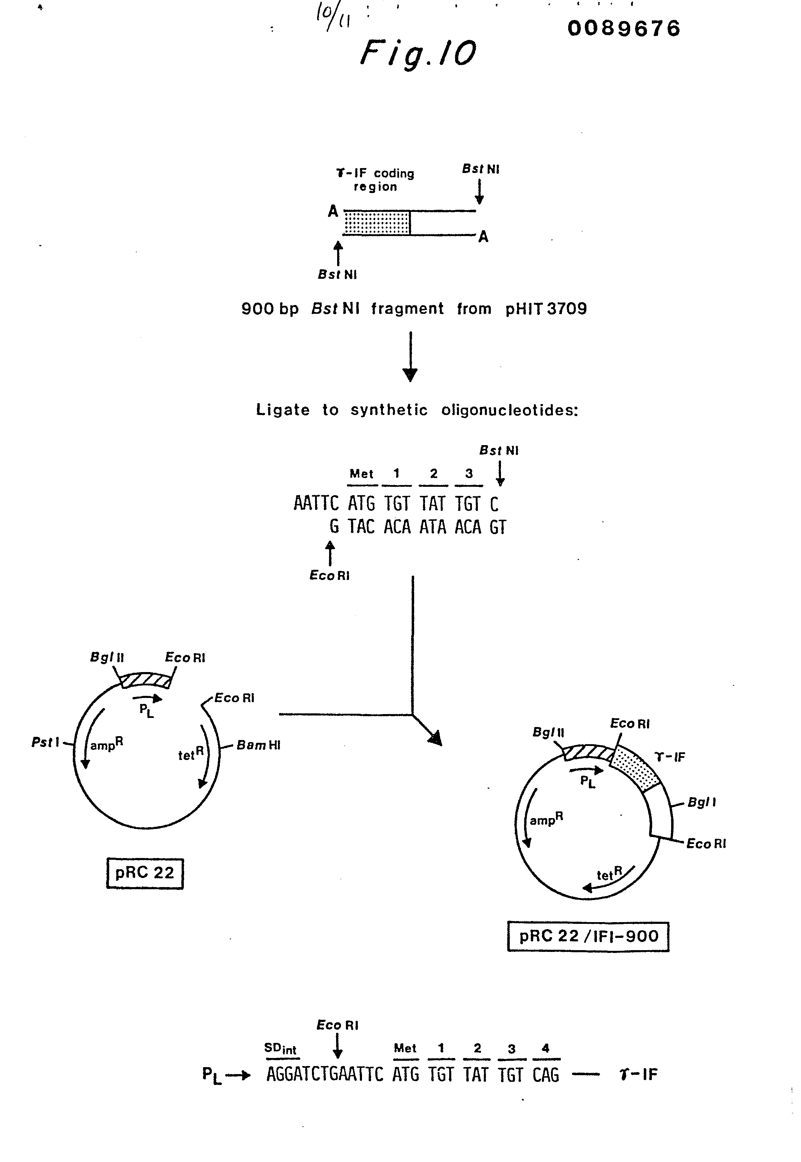

A 900 bp BstNl fragment was isolated from pHIT3709 which contains 430 bp of coding sequence for IFI and 470 bp of the 3'-noncoding region. Not present on this fragment are the sequences for the "signal" portion of IFI and the codons for the first 3 amino acids of the presumed mature protein. To restore the three missing codons and to provide an initiating Met codon, synthetic oligonucleotides were prepared and ligated to the 900 bp fragment (see Fig. 10). The synthetic segment converted both of the BstNl termini to EcoRl termini. The resulting fragment was cloned into vector pRC22 which had been restricted with EcoRl. Orientation of the insert was determined by restriction analysis with Bgl I which cuts within the 3'-noncoding region.

The pRC22 vector containing the IFI gene (denoted pRC22/IFI-900) was sequenced across the promoter-gene junction to assure that all ligation steps occurred as expected. (See Fig. 10).

The test for expression of the IFI gene, strain RRI (pRK248cIts, pRC22/IFI-900) was grown in M9-glucose media at 30°C to 3-4 × 10$^8$ cells/ml, then induced at 42°C for one hour. Prior to induction, additional glucose and casamino acids were added to 1.0 and 0.5 percent, respectively. A 10 ml sample was taken, the cells were collected by centrifugation and resuspended in 0.1 ml of 50 mM Tris (pH 7.4), 10 percent sucrose, and the suspension was quick-frozen in a dry ice/ethanol bath. The cells were thawed at 20°C, then transferred to an ice bath. NaCl was added to 100 mM, EDTA to 10 mM, spermidine to 20 mM, and lysozyme to 200 µg/ml. The mixture was kept on ice for 45 minutes, then incubated at 37°C for 2 minutes. Cell debris was removed by centrifugation and the supernatant was assayed for IFI antiviral activity on WISH cells. This initial experiment resulted in a yield of 1280 units of IFI activity per ml of cell extract.

In order to determine the kinetics of induction, the above procedure was repeated. One sample was kept at 30°C as a control, and other samples were taken after induction at 42°C for 30, 60, 90, 120, and 180 minutes. The samples were processed as described above. The results, shown in Table 2, indicate that at 30°C no activity is produced and that following induction at 42°C the amount of activity detected reaches a maximum at around 90 minutes, then gradually declines.

Table 2

| Strain | Induction conditions Temperature (°C)-Time | IFI Activity, unit/ml |
|---|---|---|
| RRl(pRK248cIts, pRC22/IFI-900) | 30° | 0 |
| " | 42° - 30'* | 120 |
| " | 42° - 60' | 120 |
| " | 42° - 90' | 320 |
| " | 42° - 120' | 160 |
| " | 42° - 180' | 40 |

*' = minutes

## Example 7

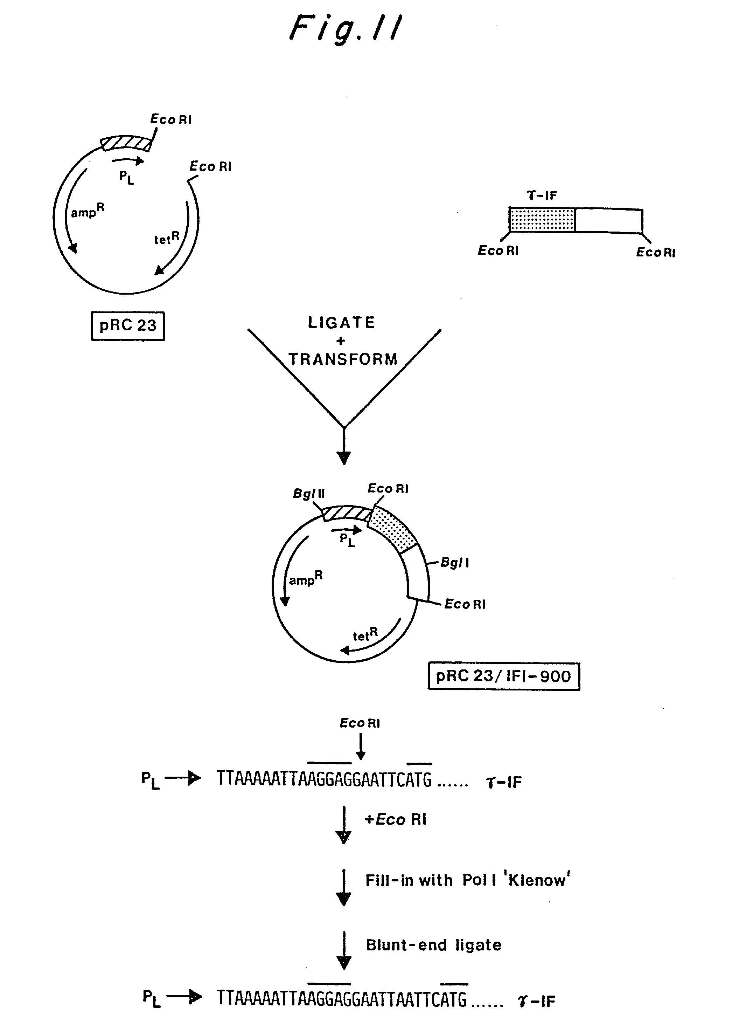

pRC22/IFI-900 DNA was further restricted with EcoRl and the 900 bp fragment containing the IFI gene was isolated and inserted into pRC23 which had been restricted with EcoRl (see Fig. 11). The resulting construction, pRC23/IFI-900, contained a RBS significantly different from that in pRC22/IFI (compare Figs. 10 and 11). To test for expression of the IFI gene, strain RRI (pRK248cIts, prC23/IFI-900) was grown in M9-glucose media at 30°C to 3-4 × $10^8$ cells/ml, then induced at 42°C for one hour. Prior to induction, additional glucose and casamino acids were added to 1.0 and 0.5 percent, respectively. A 10 ml sample was taken, the cells were collected by centrifugation and resuspended in 0.1 ml of 50 mM Tris (pH 7.4), 10 percent sucrose, and the suspension was quick-frozen in a dry ice/ethanol bath. The cells were thawed at 20°C, then transferred to an ice bath. NaCl was added to 100 mM, EDTA to 10 mM, spermidine to 20 mM, and lysozyme to 1200 µg/ml. The mixture was kept on ice for 45 minutes, then incubated at 37°C for 2 minutes. Cells debris was removed by centrifugation and the supernatant was assayed to IFI anti-viral activity on WISH cells. pRC23/IFI when used to transform E. coli strain RR1, produced about four times more activity than pRC22/IFI, as shown in Table 3.

### Table 3

| Strain | Induction conditions | IFI Activity units/ml |
|---|---|---|
| RR1(pRK248cIts, pRC22/IFI-900) | 42° - 90'* | 320 |
| RR1(pRK248cIts, pRC23/IFI-900) | 42° - 90' | 1280 |
| RR1(pRK248cIts, pRC231/IFI-900) | 42° - 90' | 640 |

*' = minutes

## Example 8

pRC23/IFI was further restricted with EcoRl under conditions that resulted in only one of two sites being cut. The resulting molecules were then treated with Pol I "Klenow" to fill-in the EcoRl termini. The blunt-ends were ligated together with $T_4$ DNA ligase and the DNA was used to transform RRI(pRK248cIts). Transformants were screened for the loss of the EcoRl site at the beginning of the IFI gene and two positives were obtained. One of these, denoted pRC231/IFI-900, was used to transform E. coli strain RR1 to express IFI. To test for expression of the IFI gene, this strain RRI PpRK248cIts, pRC231/IFI-900) was grown in M9-glucose media at 30°C to 3-4 × $10^8$ cells/ml, then induced at 42°C for one hour. Prior to induction, additional glucose and casamino acids were added to 1.0 and 0.5 percent, respectively. A 10 ml sample was taken, the cells were collected by centrifugation and resuspended in 0.1 ml of 50 mM Tris (pH 7.4), 10 percent sucrose, and the suspension was quick-frozen in a dry ice/ethanol bath. The cells were thawed at 20°C, then transferred to an ice bath. NaCl was added to 100 mM, EDTA to 10 mM, Spermidine to 20 mM, and lysozyme to 200 µg/ml. The mixture was kept on ice for 45 minutes, then incubated at 37°C for 2 minutes. Cell debris was removed by centrifugation and the supernatant was assayed for IFI anti-viral activity on WISH cells. The results are shown in Table 3.

The modification of pRC23/IFI described was designed to extend the linker region from 6 to 10 bp. In the case of IFI, a distance of 6 bp appears to be better 10 bp for expression of IFI.

### Example 9

Human immune interferon obtained in accordance with the present invention may be purified by a procedure such as the following:

All steps are preferably carried out at low temperature, preferably below room temperature, and more preferably at about 4°C. The IFI-containing material, e.g. the supernatant fluid of Example 4, can be sequentially filtered through a 3 micron and then an 0.22 micron Nalgene filter.

The next purification step utilizes a high performance weak cation-exchange liquid chromatography column. CM 200 resin, 10 m (Separation Industries) is packed into a 25 cm × 0.46 cm I.D. column, and prepared for use by cleaning with 2 M KCl and re-equilibrating with 24 mM phosphate buffer, pH 7.5, or with distilled, deionized water (which should be used throughout this procedure) the filtrate from the 0.22 microfilter is diluted with an equal volume of water and pumped at 1.5 ml/min. onto the CM column. The column is then washed with 25 mM potassium phosphate, pH 7.5, until protein in the column effluent is down to background levels, as determined by automatic monitoring of the column with fluorescamine. The column is then eluted at 0.5 ml/min with a gradient of increasing KCl (0 to 1 M) in 25 mM potassium phosphate buffer, pH 7.5.

The active fractions from the CM column are pooled and concentrated on a prewashed CF 25 filter cone (Amicon Corp.). The concentrated solution (0.5 ml or less) is then injected into a TSK 250 silica gel permeation column (30 × 0.75 cm) (Bio-Rad Laboratories), which has been pre-equilibrated, and eluted with 0.3 M KCl, 25 mM potassium phosphate, pH 7.5 at 24 ml/hr. Fractions are collected at 1 minute intervals. Interferon activity will correspond to a peak of protein. Homogeneous immune interferon may be obtained in one or more fractions at this stage, depending on the starting material and the efficiency of the earlier steps. If necessary, impure fractions can be reconcentrated and re-chromatographed on the TSK or CM column to achieve homogeneity.

The present invention has made it possible to produce human immune interferon or an equivalent thereto in an economical manner. Said interferon is useful as an antiviral or antitumor agent.

What is claimed is:

1.    A DNA which contains a base sequence of the formula:

    (5') TGT TAC TGC CAG GAC CCA TAT GTA AAA GAA

    GCA GAA AAC CTT AAG AAA TAT TTT AAT GCA GGT

    CAT TCA GAT GTA GCG GAT AAT GGA ACT CTT TTC

    TTA GGC ATT TTG AAG AAT TGG AAA GAG GAG AGT

    GAC AGA AAA ATA ATG CAG AGC CAA ATT GTC TCC

    TTT TAC TTC AAA CTT TTT AAA AAC TTT AAA GAT

    GAC CAG AGC ATC CAA AAG AGT GTG GAG ACC ATC

    AAG GAA GAC ATG AAT GTC AAG TTT TTC AAT AGC

    AAC AAA AAG AAA CGA GAT GAC TTC GAA AAG CTG

    ACT AAT TAT TCG GTA ACT GAC TTG AAT GTC CAA

    CGC AAA GCA ATA CAT GAA CTC ATC CAA GTG ATG

    GCT GAA CTG TCG CCA GCA GCT AAA ACA GGG AAG

    CGA AAA AGG AGT CAG ATG CTG TTT CGA GGT CGA

    AGA GCA TCC CAG -X. (3')

wherein X is TAA, TGA or TAG.


2.    A DNA according to Claim 1, wherein it has at the 5'-
end thereof the sequence
(5') ATG AAA TAT ACA AGT TAT ATC TTG GCT TTT CAG CTC TGC
ATC GTT TTG GGT TCT CTT GGC (3').


3.    A DNA according to Claim 1, wherein it has ATG at
the 5'-end thereof.


4.    A DNA according to any of Claims 1-3, wherein it
codes for the polypeptide:

    Cys Tyr Cys Gln Asp Pro Tyr Val Lys Glu Ala Glu

    Asn Leu Lys Lys Tyr Phe Asn Ala Gly His Ser Asp

    Val Ala Asp Asn Gly Thr Leu Phe Leu Gly Ile Leu

    Lys Asn Trp Lys Glu Glu Ser Asp Arg Lys Ile Met

    Gln Ser Gln Ile Val Ser Phe Tyr Phe Lys Leu Phe

    Lys Asn Phe Lys Asp Asp Gln Ser Ile Gln Lys Ser

    Val Glu Thr Ile Lys Glu Asp Met Asn Val Lys Phe

    Phe Asn Ser Asn Lys Lys Lys Arg Asp Asp Phe Glu

```
Lys Leu Thr Asn Tyr Ser Val Thr Asp Leu Asn Val
Gln Arg Lys Ala Ile His Glu Leu Ile Gln Val Met
Ala Glu Leu Ser Pro Ala Ala Lys Thr Gly Lys Arg
Lys Arg Ser Gln Met Lue Phe Arg Gly Arg Arg Ala
Ser Gln
```

5.     A DNA according to any of Claims 1-3, wherein it codes for a polypeptide equivalent to the immune interferon polypeptide in immunological or biological activities.

6.     A DNA according to Claims 1, 3 or 5, wherein it codes for the polypeptide:

```
Met Cys Tyr Cys Gln Asp Pro Tyr Val Lys Glu Ala
Glu Asn Leu Lys Lys Tyr Phe Asn Ala Gly His Ser
Asp Val Ala Asp Asn Gly Thr Leu Phe Leu Gly Ile
Leu Lys Asn Trp Lys Glu Glu Ser Asp Arg Lys Ile
Met Gln Ser Gln Ile Val Ser Phe Tyr Phe Lys Leu
Phe Lys Asn Phe Lys Asp Asp Gln Ser Ile Gln Lys
Ser Val Glu Thr Ile Lys Glu Asp Met Asn Val Lys
Phe Phe Asn Ser Asn Lys Lys Lys Arg Asp Asp Phe
Glu Lys Leu Thr Asn Tyr Ser Val Thr Asp Leu Asn
Val Gln Arg Lys Ala Ile His Glu Leu Ile Gln Val
Met Ala Glu Leu Ser Pro Ala Ala Lys Thr Gly Lys
Arg Lys Arg Ser Gln Met Leu Phe Arg Gly Arg Arg
Ala Ser Gln
```

7.     A DNA according to Claims 1, 2 or 5, wherein it codes for the polypeptide:

```
Met Lys Tyr Thr Ser Tyr Ile Ala Phe Gln Leu Cys
Ile Var Leu Gly Ser Leu Gly Cys Tyr Cys Gln Asp
Pro Tyr Val Lys Glu Ala Glu Asn Leu Lys Lys Tyr
Phe Asn Ala Gly His Ser Asp Val Ala Asp Asn Gly
Thr Leu Phe Leu Gly Ile Leu Lys Asn Trp Lys Glu
Glu Ser Asp Arg Lys Ile Met Gln Ser Gln Ile Val
Ser Phe Tyr Phe Lys Leu Phe Lys Asn Phe Lys Asp
Asp Gln Ser Ile Gln Lys Ser Val Glu Thr Ile Lys
```

Glu Asp Met Asn Val Lys Phe Phe Asn Ser Asn Lys

Lys Lys Arg Asp Asp Phe Glu Lys Leu Thr Asn Tyr

Ser Val Thr Asp Leu Asn Val Gln Arg Lys Ala Ile

His Glu Leu Ile Gln Val Met Ala Glu Leu Ser Pro

Ala Ala Lys Thr Gly Lys Arg Lys Arg Ser Gln Met

Leu Phe Arg Gly Arg Arg Ala Ser Gln

8.   A DNA according to any of Claims 1-7, wherein it forms a part of a recombinant DNA molecule.

9.   A DNA according to any of Claims 1-8, wherein it is linked at the site of downstream from a promoter.

10.   A method of producing a DNA containing a base sequence of the formula:

(5') TGT TAC TGC CAG GAC CCA TAT GTA AAA GAA

GCA GAA AAC CTT AAG AAA TAT TTT AAT GCA GGT

CAT TCA GAT GTA GCG GAT AAT GGA ACT CTT TTC

TTA GGC ATT TTG AAG AAT TGG AAA GAG GAG AGT

GAC AGA AAA ATA ATG CAG AGC CAA ATT GTC TCC

TTT TAC TTC AAA CTT TTT AAA AAC TTT AAA GAT

GAC CAG AGC ATC CAA AAG AGT GTG GAG ACC ATC

AAG GAA GAC ATG AAT GTC AAG TTT TTC AAT AGC

AAC AAA AAG AAA CGA GAT GAC TTC GAA AAG CTG

ACT AAT TAT TCG GTA ACT GAC TTG AAT GTC CAA

CGC AAA GCA ATA CAT GAA CTC ATC CAA GTG ATG

GCT GAA CTG TCG CCA GCA GCT AAA ACA GGG AAG

CGA AAA AGG AGT CAG ATG CTG TTT CGA GGT CGA

AGA GCA TCC CAG -X. (3')

wherein X is TAA, TGA or TAG, which comprises,

(a)   growing human immune interferon-secreting cells,

(b)   isolating from the cells a messenger RNA coding for human immune interferon,

(c)   synthesizing a single-stranded complementary DNA with the use of said messenger RNA and reverse transcriptase,

(d)     coverting said complementary DNA to a double-
        stranded DNA,

(e)     inserting said double stranded DNA into a plasmid,

(f)     transforming a host with said recombinant plasmid,

(g)     isolating a plasmid containing the objective DNA
        from the culture of said host,

(h)     if desired, excising out the cloned DNA from the
        plasmid, and

(i)     if desired, connecting said cloned DNA at the site
        of downstream from a promoter in a vehicle.


11.     A method according to Claim 10, wherein the objective
DNA codes for the polypeptide:

        Cys Tyr Cys Gln Asp Pro Tyr Val Lys Glu Ala Glu
        Asn Leu Lys Lys Tyr Phe Asn Ala Gly His Ser Asp
        Val Ala Asp Asn Gly Thr Leu Phe Leu Gly Ile Leu
        Lys Asn Trp Lys Glu Glu Ser Asp Arg Lys Ile Met
        Gln Ser Gln Ile Val Ser Phe Tyr Phe Lys Leu Phe
        Lys Asn Phe Lys Asp Asp Gln Ser Ile Gln Lys Ser
        Val Glu Thr Ile Lys Glu Asp Met Asn Val Lys Phe
        Phe Asn Ser Asn Lys Lys Lys Arg Asp Asp Phe Glu
        Lys Leu Thr Asn Tyr Ser Val Thr Asp Leu Asn Val
        Gln Arg Lys Ala Ile His Glu Leu Ile Gln Val Met
        Ala Glu Leu Ser Pro Ala Ala Lys Thr Gly Lys Arg
        Lys Arg Ser Gln Met Leu Phe Arg Gly Arg Arg Ala
        Ser Gln


12.     A method according to Claim 10, wherein the objective
DNA codes for a polypeptide equivalent to the immune
interferon polypeptide in immunological or biological
activities.


13.     A method according to Claims 10-12, wherein the
objective DNA forms a part of a recombinant DNA molecule.

14.    A host transformed with a DNA containing a base
sequence of the formula:

```
(5') TGT TAC TGC CAG GAC CCA TAT GTA AAA GAA
     GCA GAA AAC CTT AAG AAA TAT TTT AAT GCA GGT
     CAT TCA GAT GTA GCG GAT AAT GGA ACT CTT TTC
     TTA GGC ATT TTG AAG AAT TGG AAA GAG GAG AGT
     GAC AGA AAA ATA ATG CAG AGC CAA ATT GTC TCC
     TTT TAC TTC AAA CTT TTT AAA AAC TTT AAA GAT
     GAC CAG AGC ATC CAA AAG AGT GTG GAG ACC ATC
     AAG GAA GAC ATG AAT GTC AAG TTT TTC AAT AGC
     AAC AAA AAG AAA CGA GAT GAC TTC GAA AAG CTG
     ACT AAT TAT TCG GTA ACT GAC TTG AAT GTC CAA
     CGC AAA GCA ATA CAT GAA CTC ATC CAA GTG ATG
     GCT GAA CTG TCG CCA GCA GCT AAA ACA GGG AAG
     CGA AAA AGG AGT CAG ATG CTG TTT CGA GGT CGA
     AGA GCA TCC CAG -X. (3')
```
wherein X is TAA, TGA or TAG.

15.    A host according to Claim 14, wherein the DNA contains
said base sequence linked at the site of downstream from
a promoter.

16.    A host according to Claim 14 or 15, wherein said
host is _Escherichia_ _coli_ or _Bacillus_ _subtilis_.

17.    A compound of the formula:

```
Y-Cys Tyr Cys Gln Asp Pro Tyr Val Lys Glu Ala Glu
   Asn Leu Lys Lys Tyr Phe Asn Ala Gly His Ser Asp
   Val Ala Asp Asn Gly Thr Leu Phe Leu Gly Ile Leu
   Lys Asn Trp Lys Glu Glu Ser Asp Arg Lys Ile Met
   Gln Ser Gln Ile Val Ser Phe Tyr Phe Lys Leu Phe
   Lys Asn Phe Lys Asp Asp Gln Ser Ile Gln Lys Ser
   Val Glu Thr Ile Lys Glu Asp Met Asn Val Lys Phe
   Phe Asn Ser Asn Lys Lys Lys Arg Asp Asp Phe Glu
   Lys Leu Thr Asn Tyr Ser Val Thr Asp Leu Asn Val
   Gln Arg Lys Ala Ile His Glu Leu Ile Gln Val Met
```

```
    Ala Glu Leu Ser Pro Ala Ala Lys Thr Gly Lys Arg
    Lys Arg Ser Gln Met Leu Phe Arg Gly Arg Arg Ala
    Ser Gln
```
wherein Y is (a) hydrogen, (b) Met or (c) Met Lys Tyr Thr
Ser Tyr Ile Ala Phe Gln Leu Cys Ile Val Leu Gly Ser Leu
Gly.


18.     A method of producing a compound of the formula:
```
    Y-Cys Tyr Cys Gln Asp Pro Tyr Val Lys Glu Ala Glu
    Asn Leu Lys Lys Tyr Phe Asn Ala Gly His Ser Asp
    Val Ala Asp Asn Gly Thr Leu Phe Leu Gly Ile Leu
    Lys Asn Trp Lys Glu Glu Ser Asp Arg Lys Ile Met
    Gln Ser Gln Ile Val Ser Phe Tyr Phe Lys Leu Phe
    Lys Asn Phe Lys Asp Asp Gln Ser Ile Gln Lys Ser
    Val Glu Thr Ile Lys Glu Asp Met Asn Val Lys Phe
    Phe Asn Ser Asn Lys Lys Lys Arg Asp Asp Phe Glu
    Lys Leu Thr Asn Tyr Ser Val Thr Asp Leu Asn Val
    Gln Arg Lys Ala Ile His Glu Leu Ile Gln Val Met
    Ala Glu Leu Ser Pro Ala Ala Lys Thr Gly Lys Arg
    Lys Arg Ser Gln Met Leu Phe Arg Gly Arg Arg Ala
    Ser Gln
```
wherein Y is (a) hydrogen, (b) Met or (c) Met Lys Tyr Thr
Ser Tyr Ile Ala Phe Gln Leu Cys Ile Val Leu Gly Ser Leu
Gly, which comprises growing a host transformed with a
DNA containing a base sequence of the formula:
```
    (5') TGT TAC TGC CAG GAC CCA TAT GTA AAA GAA
    GCA GAA AAC CTT AAG AAA TAT TTT AAT GCA GGT
    CAT TCA GAT GTA GCG GAT AAT GGA ACT CTT TTC
    TTA GGC ATT TTG AAG AAT TGG AAA GAG GAG AGT
    GAC AGA AAA ATA ATG CAG AGC CAA ATT GTC TCC
    TTT TAC TTC AAA CTT TTT AAA AAC TTT AAA GAT
    GAC CAG AGC ATC CAA AAG AGT GTG GAG ACC ATC
    AAG GAA GAC ATG AAT GTC AAG TTT TTC AAT AGC
    AAC AAA AAG AAA CGA GAT GAC TTC GAA AAG CTG
    ACT AAT TAT TCG GTA ACT GAC TTG AAT GTC CAA
    CGC AAA GCA ATA CAT GAA CTC ATC CAA GTG ATG
```

```
GCT GAA CTG TCG CCA GCA GCT AAA ACA GGG AAG
CGA AAA AGG AGT CAG ATG CTG TTT CGA GGT CGA
AGA GCA TCC CAG -X (3')
```
wherein X is TAA, TGA or TAG, accumulating said compound
in the culture broth and recovering the same.

19.     A sterile pharmaceutical composition, comprising a
compound of the formula:

```
Y-Cys Tyr Cys Gln Asp Pro Tyr Val Lys Glu Ala Glu
    Asn Leu Lys Lys Tyr Phe Asn Ala Gly His Ser Asp
    Val Ala Asp Asn Gly Thr Leu Phe Leu Gly Ile Leu
    Lys Asn Trp Lys Glu Glu Ser Asp Arg Lys Ile Met
    Gln Ser Gln Ile Val Ser Phe Tyr Phe Lys Leu Phe
    Lys Asn Phe Lys Asp Asp Gln Ser Ile Gln Lys Ser
    Val Glu Thr Ile Lys Glu Asp Met Asn Val Lys Phe
    Phe Asn Ser Asn Lys Lys Lys Arg Asp Asp Phe Glu
    Lys Leu Thr Asn Tyr Ser Val Thr Asp Leu Asn Val
    Gln Arg Lys Ala Ile His Glu Leu Ile Gln Val Met
    Ala Glu Leu Ser Pro Ala Ala Lys Thr Gly Lys Arg
    Lys Arg Ser Gln Met Leu Phe Arg Gly Arg Arg Ala
    Ser Gln
```
wherein Y is (a) hydrogen, (b) Met or (c) Met Lys Tyr Thr
Ser Tyr Ile Ala Phe Gln Leu Cys Ile Val Leu Gly Ser Leu
Gly, and a pharmaceutically acceptable carrier.

20.     A pharmaceutical composition according to Claim 18,
wherein Y is hydrogen, and the pharmaceutically acceptable
carrier is sterile water, normal saline or human serum
albumin.

# Fig. 1

# Fig. 2

```
                                       S1
5' ACTTCTTTGGCTTAATTCTCTCGGAAACG ATG AAA TAT ACA AGT TAT ATC TTG
                                    Met Lys Tyr Thr Ser Tyr Ile Leu

                                              S20  1
GCT TTT CAG CTC TGC ATC GTT TTG GGT TCT CTT GGC TGT TAC TGC CAG
Ala Phe Gln Leu Cys Ile Val Leu Gly Ser Leu Gly Cys Tyr Cys Gln

                                                             20
GAC CCA TAT GTA AAA GAA GCA GAA AAC CTT AAG AAA TAT TTT AAT GCA
Asp Pro Tyr Val Lys Glu Ala Glu Asn Leu Lys Lys Tyr Phe Asn Ala

GGT CAT TCA GAT GTA GCG GAT AAT GGA ACT CTT TTC TTA GGC ATT TTG
Gly His Ser Asp Val Ala Asp Asn Gly Thr Leu Phe Leu Gly Ile Leu

        40
AAG AAT TGG AAA GAG GAG AGT GAC AGA AAA ATA ATG CAG AGC CAA ATT
Lys Asn Trp Lys Glu Glu Ser Asp Arg Lys Ile Met Gln Ser Gln Ile

                        60
GTC TCC TTT TAC TTC AAA CTT TTT AAA AAC TTT AAA GAT GAC CAG AGC
Val Ser Phe Tyr Phe Lys Leu Phe Lys Asn Phe Lys Asp Asp Gln Ser

                                        80
ATC CAA AAG AGT GTG GAG ACC ATC AAG GAA GAC ATG AAT GTC AAG TTT
Ile Gln Lys Ser Val Glu Thr Ile Lys Glu Asp Met Asn Val Lys Phe

                                                            100
TTC AAT AGC AAC AAA AAG AAA CGA GAT GAC TTC GAA AAG CTG ACT AAT
Phe Asn Ser Asn Lys Lys Lys Arg Asp Asp Phe Glu Lys Leu Thr Asn

TAT TCG GTA ACT GAC TTG AAT GTC CAA CGC AAA GCA ATA CAT GAA ,CTC
Tyr Ser Val Thr Asp Leu Asn Val Gln Arg Lys Ala Ile His Glu Leu

        120
ATC CAA GTG ATG GCT GAA CTG TCG CCA GCA GCT AAA ACA GGG AAG CGA
Ile Gln Val Met Ala Glu Leu Ser Pro Ala Ala Lys Thr Gly Lys Arg

                        140                         146
AAA AGG AGT CAG ATG CTG TTT CGA GGT CGA AGA GCA TCC CAG TAA TGG
Lys Arg Ser Gln Met Leu Phe Arg Gly Arg Arg Ala Ser Gln ---

TTGTCCTGCCTGCAATATTTGAATTTTAAATCTAAATCTATTTATTAATATTTAACATTATTT

ATATGGGGAATATATTTTTAGACTCATCAATCAAATAAGTATTTATAATAGCAACTTTTGTGT

AATGAAAATGAATATCTATTAATATATGTATTATTTATAATTCCTATATCCTGTGACTGTCTC

ACTTAATCCTTTGTTTTCTGACTAATTAGGCAAGGCTATGTGATTACAAGGCTTTATCTCAGG

GGCCAACTAGGCAGCCAACCTAAGCAAGATCCCATGGGTTGTGTGTTTATTTCACTTGATGAT

ACAATGAACACTTATAAGTGAAGTGATACTATCCAGTTACTGCCGGTTTGAAAATATGCCTGC

AATCTGAGCCAGTGCTTTAATGGCATGTCAGACAGAACTTGAATGTGTCAGGTGACCCTGATG

AAAACATAGCATCTCAGGAGATTTCATGCCTGGTGCTTCCAAATATTGTTGACAACTGTGACT

GTACCCAAATGGAAAGTAACTCATTTGTTAAAATTATCAATATCTAATATATATGAATAAAGT
G 3'
```

# Fig. 3

Fig.4

Fig.5

# Fig.6

λ cI857 Sam₇ DNA (dam⁻) – 49,000 bp

↓ Hinf I

Isolate 180–220 bp size class

↓ Hae II

Isolate 82 bp Hinf I – Hae II fragment

↓

Hinf I      Hae II
      Mbo I

SD_int – 82

Mbo I
↓

Hinf I ————————— TTTTGAAGAGGATCAGAAATG ——— Hae II

## Fig.7

# Fig.8

pRC 14

pRC 15

+ Bgl II
+ Eco RI

+ Bgl II
+ Eco RI

pRC 2

LIGATE
+
TRANSFORM

pRC 21

pRC 22

EcoRI

EcoRI

SD_N

SD_int

TAACTGACAGGAGAATTC

TTTTGAAGAGGATCTGAATTC

*Fig.9*

## Fig.10

T-IF coding region

BstNI

A

A

BstNI

900 bp BstNI fragment from pHIT 3709

Ligate to synthetic oligonucleotides:

BstNI

| | Met | 1 | 2 | 3 | |
|---|---|---|---|---|---|
| AATTC | ATG | TGT | TAT | TGT | C |
| G | TAC | ACA | ATA | ACA | GT |

EcoRI

pRC 22

pRC 22 / IFI-900

EcoRI

| | SD$_{int}$ | | Met | 1 | 2 | 3 | 4 | | |
|---|---|---|---|---|---|---|---|---|---|
| P$_L$ → | AGGATCTGAATTC | | ATG | TGT | TAT | TGT | CAG | —— | T-IF |

Fig.11

0089676